# EUROPÄISCHE PATENTANMELDUNG

(11) **EP 2 657 228 A1**
(43) Veröffentlichungstag der Anmeldung: **30.10.2013**
(21) Anmeldenummer: 13154273.0
(22) Anmeldetag: 06.02.2013
(51) Int. Cl.: C07D 231/14, C07D 405/04

(54) **Pyrazol-Synthese durch Kupplung von Carbonsäurederivaten und Enaminen**

(30) Priorität: 06.02.2012 DE 102012100961
(71) Anmelder: Westfälische Wilhelms-Universität Münster, 48149 Münster (DE)
(72) Erfinder: Prof. Glorius, Frank, 59387 Ascheberg (DE); Suri, Mamta, 48161 Münster (DE)
(74) Vertreter: Michalski Hüttermann & Partner Patentanwälte

(57) **Zusammenfassung**

Die vorliegende Erfindung beschreibt ein neuartiges Syntheseverfahren von Pyrazolen durch oxidative Umsetzung von Enaminen mit geeigneten N-haltigen Carbonsäurederivaten in Gegenwart von Kupferionen und 2-Picolinsäurederivaten.

## Beschreibung

Die vorliegende Erfindung bezieht sich auf das Gebiet der organischen Synthese, speziell das Gebiet der Synthese von Heterocyclen.

Heterocyclische Verbindungen spielen in der organischen Chemie, speziell bei Wirkstoffen und Pharmazeutika eine immense Rolle und die Synthese von heterocyclischen Verbindungen stellt ein wichtiges und ständig wachsendes Forschungsgebiet dar.

Eine wichtige Heterocyclenklasse stellen die Pyrazole dar. Pyrazole kommen zwar in Naturstoffen relativ selten vor, sind aber ein wichtiges Strukturelement biologisch aktiver Verbindungen, welche in einigen Fällen große wirtschaftliche Bedeutung als Pharmazeutika oder Pflanzenschutzwirkstoffe besitzen. Zwei prominente Beispiele sind der COX-2-Hemmer Celecoxib, der gegen rheumatoide Arthritis eingesetzt wird, oder das Kontaktgift Fipronil^{®}, das als GABA-Inhibitor gegen Ektoparasiten wie Flöhe, Läuse, Zecken und Milben wirksam ist.

Ein Pyrazol-Syntheseverfahren durch Kupplung von Carbonsäurederivaten und Enaminen unter oxidativen Bedingungen ist aus der WO 2011/120861 bekannt. Es stellt sich jedoch die Aufgabe, dieses Verfahren noch weiter zu verbessern.

Diese Aufgabe wird durch ein Verfahren nach Anspruch 1 gelöst. Demgemäß wird ein Verfahren zur Herstellung von Pyrazolen durch Umsetzung von Enaminen und N-haltigen Carbonsäurederivaten in Gegenwart eines Oxidationsmittels, Kupferionen sowie 2-Picolinsäure(derivaten) vorgeschlagen.

Überraschenderweise hat sich herausgestellt, dass unter diesen Bedingungen die Ausbeuten gegenüber dem aus dem Stand der Technik bekannten Verfahren meist noch deutlich gesteigert werden können.

Der Term "Umsetzung" im Sinne der vorliegenden Erfindung bedeutet insbesondere, dass die jeweiligen Substrate mit sich und den weiteren Reaktionspartnern in Kontakt gebracht werden. In der Regel geschieht dies durch Vermischen oder Suspendieren. Es sei angemerkt, dass je nach konkreter Ausführung des Verfahrens entweder die beiden Edukte vorgelegt und anschließend oxidiert wird; es kann aber auch günstig sein, ein Edukt zusammen mit dem Oxidationsmittel vorzulegen und das andere zuzugeben.

Gemäß einer bevorzugten Ausführungsform der Erfindung liegt die Reaktionstemperatur zwischen ≥0 und ≤200°C, bevorzugt zwischen ≥60 und ≤140 °C und besonders bevorzugt zwischen ≥80 und ≤120 °C.

Sowohl Druck als auch Art der Gasatmosphäre über der Reaktion sind nicht kritisch und verschiedene Gase und Drücke sind geeignet, z. B. Luft, Stickstoff, Argon, Sauerstoff, bevorzugt Luft und Stickstoff. Die Reaktion kann bei verschiedenen Drücken durchgeführt werden, bevorzugt bei Drücken von 1 bis 10 atm, besonders bevorzugt bei 1 bis 4 atm. Die Erhöhung des Drucks kann zu einer Erhöhung des Siedepunkts führen, was eine Beschleunigung der Reaktion bewirken kann.

Gegenstand der Erfindung sind weiterhin Verfahren, in denen das Reaktionsprodukt bereits unter den Reaktionsbedingungen oder durch schrittweise Variation der Reaktionsbedingungen weiterreagiert, z. B. durch eine Verseifung einer Estergruppe, eine Decarboxylierungsreaktion oder eine Hydrierung.

Die Aufarbeitung der Reaktionsgemische und die Reinigung der Produkte sind üblicherweise weniger kritisch und richten sich nach den jeweiligen physikalischen Eigenschaften der erzeugten Produkte und Nebenprodukte. Bevorzugte Aufarbeitungs- und Reinigungsmethoden sind Destillation, Sublimation, Kristallisation, Chromatographie, Filtration und Extraktion. Dadurch, dass Hydrazine vermieden werden, ist eine Aufreinigung im Vergleich zu den bisherigen Reaktionsverfahren meist einfacher und unter Erhalt eines Produktes in größerer Reinheit möglich.

Gemäß einer bevorzugten Ausführungsform der vorliegenden Erfindung beträgt das Verhältnis von Enaminen zu N-haltigen-Carbonsäurederivaten von (mol N-haltige Carbonsäurederivate:mol Enamin) ≥0.2:1 bis ≤150:1, bevorzugt ≥1:1 bis ≤100:1, sowie am meisten bevorzugt von ≥2:1 bis ≤20:1. Der Überschuss an N-haltigem Carbonsäurederivat kann u.a. dadurch erfolgen, dass die Reaktion im N-haltigem Carbonsäurederivat als Lösemittel erfolgt. In diesem Fall sind bevorzugte Verbindungen insbesondere Acetonitril, Propionitril, Isobutyronitril, Butyronitril, Valeronitril, Capronitril, Heptansäurenitril, Octansäurenitril, Nonansäurenitril, Benzonitril, sowie ihrer mono- oder mehrfach fluorierten, geradkettigen und verzweigten Derivate.

Gemäß einer bevorzugten Ausführungsform der vorliegenden Erfindung findet die Reaktion in einem Lösemittel statt, ausgewählt aus der Gruppe enthaltend Pentan, Hexan, Heptan, Octan, Petrolether, Toluol, Xylole, Chlorbenzol, o-Dichlorbenzol, Ethylacetat, Tetrahydrofuran, Diethylether, Methyltertbutylether, 1,4-Dioxan, N,N-Dimethylformamid, Dimethylsulfoxid, 1,2-Dichlorethan, N,N-Dimethylacetamid, Dimethoxyethan oder Carbonsäuren wie Ameisensäure, Essigsäure, Trifluoressigsäure, Chloressigsäure, Trichloressigsäure, Propionsäure, Buttersäure, oder Alkohole wie Methanol, Ethanol, Propanol, Isopropanol, Butanol, t-Butanol oder Phenol, bevorzugt Ethylacetat, Tetrahydrofuran, Diethylether, Methyltertbutylether, 1,4-Dioxan, N,N-Dimethylformamid, Dimethylsulfoxid, 1,2-Dichlorethan, N,N-Dimethylacetamid, t-Butanol, Chlorbenzol, Toluol, Dimethoxyethan, Hexan, o-Dichlorbenzol, besonders bevorzugt 1,4-Dioxan, N,N-Dimethylformamid, Dimethylsulfoxid, 1,2-Dichlorethan, N,N-Dimethylacetamid, t-Butanol und Chlorbenzol oder Mischungen daraus.

Gemäß einer bevorzugten Ausführungsform der vorliegenden Erfindung sind die N-haltigen Carbonsäurederivate ausgewählt aus der Gruppe enthaltend Nitrile, Carbonsäureamide, Amidine, Imidate, Imidoylchloride, abgeleitete protonierte Salze dieser Verbindungen sowie Mischungen daraus.

Bevorzugt werden als Enamine Verbindungen der folgenden Struktur eingesetzt: 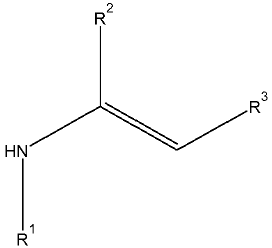 wobei R¹, R² und R³ unabhängig voneinander ausgewählt sind aus der Gruppe enthaltend Wasserstoff, Hydroxyl, Halogen, Pseudohalogen, Formyl, Carboxy- und/oder Carbonyl derivaten, Alkyl, langkettiges Alkyl, Alkoxy, langkettiges Alkoxy, Cycloalkyl, Halogenalkyl, Aryl, Arylene, Halogenaryl, Heteroaryl, Heteroarylene, Heterocycloalkylene, Heterocycloalkyl, Halogenheteroaryl, Alkenyl, Halogenalkenyl, Alkinyl, Halogenalkinyl, Keto, Ketoaryl, Halogenketoaryl, Ketoheteroaryl, Ketoalkyl, Halogenketoalkyl, Ketoalkenyl, Halogenketoalkenyl, Phosphoalkyl, Phosphonate, Phosphate, Phosphin, Phosphinoxid, Phosphoryl, Phosphoaryl, Sulphonyl, Sulphoalkyl, Sulphoarenyl, Sulphonate, Sulphate, Sulphone, Amine, Polyether, Silylalkyl, Silylalkyloxy, wobei bei geeigneten Resten eine oder mehrere nicht-benachbarte CH₂-Gruppen unabhängig voneinander durch -O-, -S-, -NH-, - NR°-, -SiR°R°°-, -CO-, -COO-, -OCO-, -OCO-O-, -SO₂- -S-CO-, -CO-S-, -CY1=CY2 oder - C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)

Bevorzugt werden als N-haltigen Carbonsäurederivate Verbindung mit folgenden Strukturen eingesetzt: 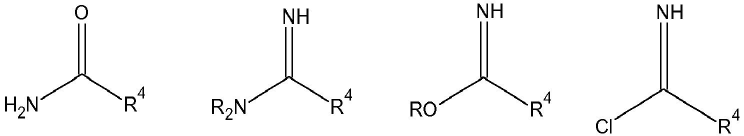 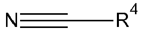 wobei R ausgewählt ist aus Alkyl, Aryl, Halogenalkyl und R⁴ ausgewählt ist aus der Gruppe enthaltend Wasserstoff, Hydroxyl, Halogen, Pseudohalogen, Formyl, Carboxy- und/oder Carbonyl derivaten, Alkyl, langkettiges Alkyl, Alkoxy, langkettiges Alkoxy, Cycloalkyl, Halogenalkyl, Aryl, Arylene, Halogenaryl, Heteroaryl, Heteroarylene, Heterocycloalkylene, Heterocycloalkyl, Halogenheteroaryl, Alkenyl, Halogenalkenyl, Alkinyl, Halogenalkinyl, Keto, Ketoaryl, Halogenketoaryl, Ketoheteroaryl, Ketoalkyl, Halogenketoalkyl, Ketoalkenyl, Halogenketoalkenyl, Phosphoalkyl, Phosphonate, Phosphate, Phosphin, Phosphinoxid, Phosphoryl, Phosphoaryl, Sulphonyl, Sulphoalkyl, Sulphoarenyl, Sulphonate, Sulphate, Sulphone, Amine, Polyether, Silylalkyl, Silylalkyloxy, wobei bei geeigneten Resten eine oder mehrere nicht-benachbarte CH₂-Gruppen unabhängig voneinander durch -O-, -S-, -NH-, - NR°-, -SiR°R°°-, -CO-, -COO-, -OCO-, -OCO-O-, -SO₂- -S-CO-, -CO-S-, -CY1=CY2 oder - C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
allgemeine Gruppendefinition: Innerhalb der Beschreibung und den Ansprüchen werden allgemeine Gruppen, wie z.B: Alkyl, Alkoxy, Aryl etc. beansprucht und beschrieben. Wenn nicht anders beschrieben, werden bevorzugt die folgenden Gruppen innerhalb der allgemein beschriebenen Gruppen im Rahmen der vorliegenden Erfindung verwendet:
alkyl: lineare und verzweigte C1-C8-Alkyle,
langkettige Alkyle: lineare und verzweigte C5-C20 Alkyle
alkenyl: C2-C6-alkenyl,
cycloalkyl: C3-C8-cycloalkyl,
alkoxy: C1-C6-alkoxy,
langkettig Alkoxy: lineare und verzweigte C5-C20 Alkoxy
alkylene: ausgewählt aus der Gruppe enthaltend:
methylene; 1,1-ethylene; 1,2-ethylene; 1,1-propylidene; 1,2-propylene; 1,3- propylene; 2,2-propylidene; butan-2-ol-1,4-diyl; propan-2-ol-1,3-diyl; 1, 4-butylene; cyclohexane-1,1-diyl; cyclohexan-1,2-diyl; cyclohexan-1,3- diyl; cyclohexan-1,4-diyl; cyclopentane-1,1-diyl, cyclopentan-1,2-diyl; und cyclopentan-1,3-diyl,
aryl: ausgewählt aus Aromaten mit einem Molekulargewicht unter 300Da.
arylene: ausgewählt aus der Gruppe enthaltend: 1,2-phenylene; 1,3- phenylene; 1,4-phenylene; 1,2-naphtalenylene; 1,3-naphtalenylene; 1,4- naphtalenylene; 2,3-naphtalenylene; 1-hydroxy-2,3-phenylene; 1-hydroxy-2,4- phenylene; 1-hydroxy-2,5- phenylene; und 1-hydroxy-2,6-phenylene,
heteroaryl: ausgewählt aus der Gruppe enthaltend: pyridinyl; pyrimidinyl; pyrazinyl; triazolyl; pyridazinyl; 1,3,5-triazinyl; chinoninyl; isochinoninyl; chinoxalinyl; imidazolyl; pyrazolyl; benzimidazolyl; thiazolyl; oxazolidinyl; pyrrolyl; thiophenyl; carbazolyl; indolyl; und isoindolyl, wobei das Heteroaryl mit der Verbindung über jedes Atom im Ring des ausgewählten Heteroaryls verbunden sein kann.
heteroarylene: ausgewählt aus der Gruppe enthaltend: pyridindiyl; quinolindiyl; pyrazodiyl; pyrazoldiyl; triazolediyl; pyrazindiyl, thiophendiyl; und imidazolediyl, wobei das heteroarylene als Brücke in der Verbindung über ein beliebiges Atom im Ring des ausgewählten Heteroaryls fungiert, speziell bevorzugt sind: pyridin-2, 3-diyl; pyridin-2,4-diyl; pyridin-2,5-diyl; pyridin-2,6-diyl; pyridin-3,4- diyl; pyridin-3,5-diyl; quinolin-2,3-diyl; quinolin-2,4-diyl; quinolin-2, 8-diyl; isoquinolin-1,3-diyl; isoquinolin-1,4-diyl; pyrazol-1,3-diyl; pyrazol-3,5- diyl; triazole-3,5-diyl; triazole-1,3-diyl; pyrazin-2,5-diyl; und imidazole-2,4-diyl, thiophen-2,5-diyl, thiophen-3,5-diyl; ein -Cl-C6-heterocycloalkyl, ausgewählt aus der Gruppe enthaltend: piperidinyl; piperidine; 1,4-piperazine, tetrahydrothiophene; tetrahydrofuran; 1,4,7-triazacyclononane; 1,4,8,11-tetraazacyclotetradecane; 1,4,7,10,13-pentaazacyclopentadecane; 1,4-diaza- 7-thia-cyclononane; 1,4- diaza-7-oxa-cyclononane; 1,4,7,10-tetraazacyclododecane; 1,4-dioxane; 1,4, 7-trithia-cyclononane; pyrrolidine; und
tetrahydropyran, wobei das Heteroaryl mit dem Cl-C6-Alkyl über jedes Atom im Ring des ausgewählten Heteroaryls verbunden sein kann.
heterocycloalkylene: ausgewählt aus der Gruppe enthaltend: piperidin-1,2- ylene; piperidin-2,6-ylene; piperidin-4,4-ylidene; 1,4-piperazin-1,4-ylene; 1,4-piperazin-2,3-ylene; 1,4-piperazin-2,5-ylene; 1,4-piperazin-2,6-ylene; 1,4-piperazin- 1,2-ylene; 1,4-piperazin-1,3-ylene; 1,4-piperazin-1,4-ylene; tetrahydrothiophen-2,5-ylene; tetrahydrothiophen-3,4-ylene; tetrahydrothiophen-2,3-ylene; tetrahydrofuran-2,5-ylene; tetrahydrofuran- 3,4-ylene; tetrahydrofuran-2,3-ylene, pyrrolidin-2,5-ylene; pyrrolidin-3,4-ylene; pyrrolidin-2,3-ylene; pyrrolidin-1,2-ylene; pyrrolidin-1,3-ylene; pyrrolidin-2,2-ylidene; 1,4,7-triazacyclonon-1,4-ylene; 1,4,7- triazacyclonon-2,3-ylene; 1,4,7-triazacyclonon-2,9-ylene; 1,4,7-triazacyclonon-3,8-ylene; 1,4,7-triazacyclonon-2,2- ylidene; 1,4,8,11-tetraazacyclotetradec-1,4-ylene; 1,4,8,11- tetraazacyclotetradec-1,8-ylene; 1,4,8,11-tetraazacyclotetradec-2,3-ylene; 1,4,8,11-tetraazacyclotetradec-2,5-ylene; 1,4,8,11- tetraazacyclotetradec-1,2-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-1,2- ylene; 1,4,7,10-tetraazacyclododec-2,3- ylene; 1,4,7,10-tetraazacyclododec-2,2-ylidene; 1,4,7,10,13 3 pentaazacyclopentadec-1,4-ylene; 1,4,7,10,13-pentaazacyclopentadec-1,7-ylene; 1,4,7,10,13-pentaazacyclopentadec-2,3- ylene; 1,4,7,10,13-pentaazacyclopentadec-1,2-ylene; 1,4,7,10, 13-pentaazacyclopentadec-2,2-ylidene; 1,4-diaza-7-thia-cyclonon- 1,4-ylene; 1,4-diaza-7-thia-cyclonon-1,2-ylene; 1,4-diaza-7thia-cyclonon- 2,3-ylene; 1,4-diaza-7-thia-cyclonon-6,8-ylene; 1,4-diaza-7-thia-cyclonon- 2,2-ylidene; 1,4-diaza-7-oxacyclonon-1,4-ylene; 1,4-diaza-7-oxa-cyclonon- 1,2-ylene; l,4diaza-7-oxa-cyclonon-2,3-ylene; 1,4-diaza-7-oxa-cyclonon-6, 8-ylene; 1,4-diaza-7-oxa-cyclonon-2,2-ylidene; 1,4-dioxan-2,3-ylene; 1,4- dioxan-2,6-ylene; 1,4-dioxan-2,2-ylidene; tetrahydropyran-2,3-ylene; tetrahydropyran-2,6-ylene; tetrahydropyran-2,5-ylene; tetrahydropyran-2,2- ylidene; 1,4,7-trithia-cyclonon-2,3-ylene; 1,4,7-trithia-cyclonon-2,9- ylene; und 1,4,7-trithia-cyclonon-2,2-ylidene,
heterocycloalkyl: ausgewählt aus der Gruppe enthaltend: pyrrolinyl; pyrrolidinyl; morpholinyl; piperidinyl; piperazinyl; hexamethylene imine; 1,4-piperazinyl; tetrahydrothiophenyl; tetrahydrofuranyl; 1,4,7- triazacyclononanyl; 1,4,8,11-tetraazacyclotetradecanyl; 1,4,7,10,13- pentaazacyclopentadecanyl; 1,4-diaza-7-thiacyclononanyl; 1,4-diaza-7-oxa- cyclononanyl; 1,4,7,10-tetraazacyclododecanyl; 1,4-dioxanyl; 1,4,7- trithiacyclononanyl; tetrahydropyranyl; und oxazolidinyl, wobei das Heterocycloalkyl mit der Verbindung über jedes Atom im Ring des ausgewählten Heterocycloalkyls verbunden sein kann.
amine: die Gruppe -N(R)2 wobei jedes R unabhängig ausgewählt ist aus: Wasserstoff; Cl-C6-alkyl; C1-C6-alkyl-C6H5, und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
halogen: ausgewählt aus der Gruppe enthaltend: F; Cl; Br und I,
halogenalkyl: ausgewählt aus der Gruppe enthaltend mono, di, tri-, poly und perhalogenated lineare und verzweigte C1-C8-alkyl
pseudohalogen: ausgewählt aus der Gruppe enthaltend -CN, -SCN, -OCN, N3, -CNO, -SeCN
sulphonate: die Gruppe -S(O)2OR, wobei R ausgewählt ist aus: Wasserstoff; Cl- C6-alkyl; phenyl; C1-C6-alkyl-C6H5; Li; Na; K; Cs; Mg; und Ca,
sulphate: die Gruppe -OS(O)2OR, wobei R ausgewählt ist aus: Wasserstoff; Cl- C6-alkyl; phenyl; C1-C6-alkyl-C6H5; Li; Na; K; Cs; Mg; und Ca,
sulphone: die Gruppe -S(O)2R, wobei R ausgewählt ist aus: Wasserstoff; C1-C6- alkyl; phenyl; C1-C6-alkyl-C6H5 und amine (to give sulphonamide) ausgewählt aus der Gruppe: - NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
carboxylat : die Gruppe -C(O)OR, wobei R ausgewählt ist aus: Wasserstoff; C1-C6-alkyl; phenyl; C1-C6-alkyl-C6H5; Li; Na; K; Cs; Mg; und Ca,
carbonyl : die Gruppe -C(O)R, wobei R ausgewählt ist aus: Wasserstoff; C1-C6-alkyl; phenyl; C1-C6-alkyl-C6H5 und amine (to give amide) ausgewählt aus der Gruppe: -NR'2, wobei jedes R' unabhängig ausgewählt ist aus: Wasserstoff; Cl- C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
phosphonate: die Gruppe -P(O) (OR) 2, wobei jedes R unabhängig ausgewählt ist aus: Wasserstoff; C1-C6-alkyl; phenyl; C1-C6-alkyl-C6H5; Li; Na; K; Cs; Mg; und Ca,
phosphate: die Gruppe -OP(O)(OR)2, wobei jedes R unabhängig ausgewählt ist aus: Wasserstoff; C1-C6-alkyl; phenyl; C1-C6-alkyl-C6H5; Li; Na; K; Cs; Mg; und Ca,
phosphine: die Gruppe -P(R)2, wobei jedes R unabhängig ausgewählt ist aus: Wasserstoff; C1-C6-alkyl; phenyl; und C1-C6-alkyl-C6H5,
Phosphinoxid: die Gruppe -P (O) R2, wobei R unabhängig ausgewählt ist aus: Wasserstoff; C1-C6-alkyl; phenyl; und C1-C6-alkyl-C6H5; und amine (to give phosphonamidate) ausgewählt aus der Gruppe: -NR'2, wobei jedes R' unabhängig ausgewählt ist aus: Wasserstoff; C1-C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet.
polyether: ausgewählt aus der Gruppe enthaltend -(O-CH₂-CH(R))ₙ-OH und -(O-CH₂-CH(R))ₙ-H wobei R unabhängig ausgewählt ist aus: Wasserstoff, alkyl, aryl, halogen und n ist von 1 to 250
silylalkyl: die Gruppe -SiR₃, wobei jedes R unabhängig voneinander ausgewählt ist aus: Wasserstoff; C1-C6- alkyl; phenyl; C1-C6-alkyl-C6H5 und amine (to give sulphonamide) ausgewählt aus der Gruppe: -NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
silylalkyloxy: die Gruppe -OSiR₃ , wobei jedes R unabhängig voneinander ausgewählt ist aus: Wasserstoff; C1-C6- alkyl; phenyl; C1-C6-alkyl-C6H5 und amine (to give sulphonamide) ausgewählt aus der Gruppe: -NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet

Soweit nicht anders erwähnt, sind die folgenden Gruppen mehr bevorzugte Gruppen innerhalb der allgemeinen Gruppendefinition:
alkyl: lineare und verzweigte C1-C6-alkyl,
langkettige Alkyle: lineare und verzweigte C5-C10 alkyl, vorzugsweise C6-C8 alkyle
alkenyl: C3-C6-alkenyl,
cycloalkyl: C6-C8-cycloalkyl,
alkoxy: C1-C4-alkoxy,
langkettig Alkoxy: lineare und verzweigte C5-C10 alkoxy, vorzugsweise lineare C6-C8 alkoxy
alkylene: ausgewählt aus der Gruppe enthaltend: methylene; 1,2-ethylene; 1,3-propylene; butan-2-ol-1,4-diyl; 1,4-butylene; cyclohexane-1,1-diyl, cyclohexan-1,2-diyl; cyclohexan-1,4-diyl; cyclopentane-1,1-diyl, und cyclopentan-1,2-diyl,
aryl: ausgewählt aus der Gruppe enthaltend: phenyl; biphenyl; naphthalenyl; anthracenyl; und phenanthrenyl,
arylene: ausgewählt aus der Gruppe enthaltend: 1,2-phenylene; 1,3- phenylene; 1,4-phenylene; 1,2-naphtalenylene; 1,4-naphtalenylene; 2,3- naphtalenylene und 1-hydroxy-2,6-phenylene,
heteroaryl: ausgewählt aus der Gruppe enthaltend:
   pyridinyl; pyrimidinyl; chinoninyl; pyrazolyl; triazolyl; isochinoninyl; imidazolyl; und oxazolidinyl, wobei das Heteroaryl mit der Verbindung über jedes Atom im Ring des ausgewählten Heteroaryls verbunden sein kann,
heteroarylene: ausgewählt aus der Gruppe enthaltend: pyridin 2,3-diyl; pyridin-2,4-diyl; pyridin-2,6-diyl; pyridin-3,5-diyl; quinolin-2,3-diyl; quinolin-2,4-diyl; isoquinolin-1,3-diyl; isoquinolin-1,4-diyl; pyrazol-3,5-diyl; und imidazole-2,4-diyl,
heterocycloalkyl: ausgewählt aus der Gruppe enthaltend:
   pyrrolidinyl; morpholinyl; piperidinyl; piperidinyl; 1,4 piperazinyl; tetrahydrofuranyl; 1,4,7-triazacyclononanyl; 1,4,8,11-tetraazacyclotetradecanyl; 1,4,7,10,13-pentaazacyclopentadecanyl; 1,4,7,10-tetraazacyclododecanyl; und piperazinyl, wobei das Heteroaryl mit der Verbindung über jedes Atom im Ring des ausgewählten Heteroaryls verbunden sein kann
   heterocycloalkylene: ausgewählt aus der Gruppe enthaltend:
      piperidin-2,6-ylene; piperidin-4,4-ylidene; 1,4-piperazin-1,4-ylene; 1,4-piperazin-2,3-ylene; 1,4-piperazin-2,6-ylene; tetrahydrothiophen-2,5-ylene; tetrahydrothiophen-3,4-ylene; tetrahydrofuran-2,5-ylene; tetrahydrofuran-3,4-ylene; pyrrolidin-2,5-ylene; pyrrolidin-2,2-ylidene; 1,4,7-triazacyclonon-1,4- ylene; 1,4,7-triazacyclonon-2,3-ylene; 1,4,7-triazacyclonon-2,2-ylidene; 1,4,8,11- tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-2,3-ylene; 1,4,7,10-tetraazacyclododec-2,2-ylidene; 1,4,7,10,13- pentaazacyclopentadec-l,4-ylene; 1,4,7,10,13-pentaazacyclopentadec-1,7-ylene; 1,4-diaza-7-thia-cyclonon-1,4 ylene; 1,4-diaza-7-thia-cyclonon-2,3-ylene; 1,4-diaza-7-thiein cyclonon-2,2-ylidene; 1,4-diaza-7-oxa-cyclonon-1,4-ylene; 1,4 diaza-7-oxa-cyclonon-2,3-ylene;l,4-diaza-7-oxa-cyclonon-2,2- ylidene; 1,4-dioxan-2,6-ylene; 1,4-dioxan-2,2-ylidene; tetrahydropyran-2,6-ylene; tetrahydropyran-2,5-ylene; und tetrahydropyran- 2,2-ylidene, ein -C1-C6-alkyl-heterocycloalkyl, wobei das Heterocycloalkyl ausgewählt aus der Gruppe enthaltend: piperidinyl; 1,4-piperazinyl; tetrahydrofuranyl; 1,4,7- triazacyclononanyl; 1,4,8,11-tetraazacyclotetradecanyl; 1,4,7,10,13-pentaazacyclopentadecanyl; 1,4,7,10-tetraazacyclododecanyl; und pyrrolidinyl, wobei das Heterocycloalkyll mit der Verbindung über jedes Atom im Ring des ausgewählten Heterocycloalkyls verbunden sein kann
amine: die Gruppe -N (R) 2, wobei jedes R unabhängig ausgewählt ist aus: Wasserstoff; C1-C6-alkyl; und benzyl,
halogen: ausgewählt aus der Gruppe enthaltend: F und C1,
sulphonate: die Gruppe -S(O)2OR, wobei R ausgewählt ist aus: Wasserstoff; Cl- C6-alkyl; Na; K; Mg; und Ca,
sulphate: die Gruppe -OS(O)2OR, wobei R ausgewählt ist aus: Wasserstoff; Cl- C6-alkyl; Na; K; Mg; und Ca,
sulphone: die Gruppe -S(O)2R, wobei R ausgewählt ist aus: Wasserstoff; C1-C6- alkyl; benzyl und amine ausgewählt aus der Gruppe: -NR'2, wobei jedes R' unabhängig ausgewählt ist aus:
   Wasserstoff; C1-C6-alkyl; und benzyl,
carboxylat: die Gruppe -C(O)OR, wobei R ausgewählt ist aus Wasserstoff; Na; K; Mg; Ca; C1-C6-alkyl; und benzyl,
carbonyl : die Gruppe: -C(O)R, wobei R ausgewählt ist aus: Wasserstoff; C1-C6-alkyl; benzyl und amine ausgewählt aus der Gruppe: -NR'2, wobei jedes R' unabhängig ausgewählt ist aus: Wasserstoff; C1-C6-alkyl; und benzyl,
phosphonate: die Gruppe -P(O) (OR)2, wobei jedes R unabhängig ausgewählt ist aus: Wasserstoff; C1-C6-alkyl; benzyl; Na; K; Mg; und Ca,
phosphate: die Gruppe -OP(O) (OR)2, wobei jedes R unabhängig ausgewählt ist aus: Wasserstoff; C1-C6-alkyl; benzyl; Na; K; Mg; und Ca,
phosphine: die Gruppe -P(R)2, wobei jedes R unabhängig ausgewählt ist aus: Wasserstoff; C1-C6-alkyl; und benzyl,
Phosphinoxid: die Gruppe -P(O)R2, wobei R unabhängig ausgewählt ist aus: Wasserstoff; C1-C6-alkyl; benzyl und amine ausgewählt aus der Gruppe: -NR'2, wobei jedes R' unabhängig ausgewählt ist aus: Wasserstoff; C1-C6-alkyl; und benzyl.
polyether: ausgewählt aus der Gruppe enthaltend-(O-CH₂-CH(R))ₙ-OH und -(O-CH₂-CH(R))ₙ-H wobei R unabhängig ausgewählt ist aus: Wasserstoff, methyl, halogen und n ist von 5 bis 50, bevorzugt 10 bis 25.
M, Mₙ (n ist eine ganze Zahl) : Metalle, wobei zwei Metalle M unabhängig voneinander ausgewählt sind, wenn nicht anders angezeigt.

Bevorzugt werden Enamine der folgenden Struktur verwendet: 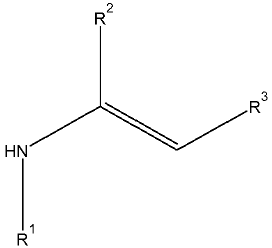 wobei R¹ und R² wie oben beschrieben sind, R³ aber eine elektronenziehende Gruppe enthält, d.h. ausgewählt ist aus der Gruppe enthaltend Formyl, Carboxy- und/oder Carbonylderivate, Keto, Ketoaryl, Halogenketoaryl, Ketoheteroaryl, Ketoalkyl, Halogenketoalkyl, Ketoalkenyl, Halogenketoalkenyl, Phosphoalkyl, Phosphonate, Phosphate, Phosphin, Phosphinoxid, Phosphoryl, Phosphoaryl, Sulphonyl, Sulphoalkyl, Sulphoarenyl, Sulphonate, Sulphate, Sulphone, Amine, Polyether, Silylalkyl, Silylalkyloxy, wobei bei geeigneten Resten eine oder mehrere nicht-benachbarte CH₂-Gruppen unabhängig voneinander durch -O-, -S-, -NH-, - NR°-, -SiR°R°°-, -CO-, -COO-, -OCO-, -OCO-O-, -SO₂-, -S-CO-, -CO-S-, -CY1=CY2 oder - C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Crruppen werden wie CH₂-Crruppen im Sinne von CH₂-H verstanden.)

Verbindungen dieses Typs haben sich in der Praxis als besonders günstig herausgestellt, da zum einen die so erhaltenen Enamine meist deutlich stabiler sind und zum anderen oftmals die Reaktivität größer ist.

Es sei an dieser Stelle angemerkt, dass die erfindungsgemäßen Enamine je nach Ausführungsform entweder in Substanz eingesetzt werden oder während der Umsetzung in *situ* hergestellt werden. Dies kann (je nach Ausführungsform) durch Umsetzung von geeigneten Aminen mit Ketonen (ggf. unter Verwendung wasserentziehender Substanzen wie etwa TiCl₄) oder (wenn ein Enamin der obigen Struktur mit einer elektronenziehenden Gruppe verwendet wird) durch Michael-Addition eines Amins an ein Alkin erfolgen.

Ebenso sei an dieser Stelle angemerkt, dass - falls Nitrile als N-haltigen Carbonsäurederivate gewählt werden - diese entweder in Substanz eingesetzt werden können oder aus anderen Carbonsäurederivaten oder geeigneten Vorläufersubstanzen *in situ* während der Reaktion hergestellt werden können. Geeignete Vorläufersubstanzen sind insbesondere diese, die bei wasserentziehenden Bedingungen Nitrile ergeben. In diesem Fall kann - falls gewünscht - sowohl das Enamin wie das Nitril *in situ* während des Verfahrens entstehen.

Wie beschrieben, wird das Verfahren in Gegenwart von Kupferionen durchgeführt. Besonders bevorzugt sind Kupfer(II)-Salze. Für den Fall, dass neben den Kupfer-Ionen noch ein Oxidationsmittel vorhanden ist (vgl. unten) sind bevorzugte Mengenbereiche ≥1% bis ≤20%, noch bevorzugt ≥2% bis ≤10% (bezogen auf das Enamin in mol/mol, vor Reaktionsbeginn).

Gemäß einer bevorzugten Ausführungsform ist das Oxidationsmittel ausgewählt aus der Gruppe enthaltend Sauerstoff (bzw. Luft), Ozon, Hypohalogenide, insbesondere Hypochlorit, Peroxide, Metalle bzw. Metallsalze oder Mischungen daraus.

Besonders bevorzugt sind Metallsalze, dabei insbesondere Cu(II)-Salze, bevorzugt Cu(OAc)₂ oder Cu(OAc)₂-Hydrat.

Das Oxidationsmittel wird bevorzugt in einem Verhältnis (bezogen auf das Enamin) von ≥1.5:1 bis ≤30:1 (mol Oxidationsmittel : mol Enamin) eingesetzt, noch bevorzugt ist ein Verhältnis von ≥2:1 bis ≤10:1, noch bevorzugt ≥2.5:1 bis ≤3:1

Besonders bevorzugt ist der Einsatz von Cu(II)-Salzen, insbesondere aus dem Grund, dass das Kupfer gleichzeitig als Lewis-Säure agieren kann und somit oxidierend wie katalytisch aktiv wirkt. Demgemäß ist es bevorzugt, dass die Umsetzung in Gegenwart von Cu(II)-Salzen durchgeführt wird und das Verhältnis von Enamin zu Cu(II)-Salz von ≥1.5:1 bis ≤30:1 (mol Cu(II)-Salz :mol Enamin, vor Reaktionsbeginn) beträgt.

Die vorliegende Erfindung ist aber nicht darauf beschränkt, sondern es kann genauso ein Oxidationsmittel neben den Kupfer-Ionen eingesetzt werden.

Wie beschrieben, findet die Reaktion in Gegenwart von 2-Picolinsäure(derivaten) statt. Unter "2-Picolinsäure(derivaten)" werden dabei 2-Picolinsäure selbst sowie Alkyl- und Halogenderivate der 2-Picolinsäure bzw. Mischungen dieser Verbindungen verstanden.

Bevorzugt beträgt dabei das Verhältnis von 2-Picolinsäure(derivaten) zu Enamin (vor Reaktionsbeginn) von ≥0.01% bis ≤10% (mol/mol), noch bevorzugt ≥0.05% bis ≤8%, sowie am meisten bevorzugt ≥1% bis ≤5%.

Ohne darauf beschränkt zu sein, wird vermutet, dass das Kupfer mit der 2-Picolinsäure sowie dem Enamin einen Chelat-Komplex bildet, an welchen sich dann das Nitril anlagert. In einer (zumindest formalen) Redoxreaktion entsteht dann das Pyrazol unter Reduktion des Kupfers. Dieses wird dann ggf. - so Kupfer unterstöchiometrisch eingesetzt wird - dann wieder oxidiert so dass ein neuer Reaktionszyklus stattfinden kann. Die 2-Picolinsäure ist aus sterischen wie elektronischen Gründen für die Bildung dieses Komplexes besonders günstig.

Für den Fall, dass Sauerstoff (bzw. Luft) als Oxidationsmittel eingesetzt wird, hat sich in der Praxis herausgestellt, dass die Ausbeuten durch Zusatz einer in α-Stellung tertiären oder sekundären Carbonsäure zusätzlich zu der 2-Picolinsäure nochmals erhöht werden können. Dies ist somit eine bevorzugte Ausführungsform der vorliegenden Erfindung. Bevorzugt sind dabei α-tertiäre Carbonsäuren wie z.B. Pivalinsäure. Das Konzentration beträgt dabei (in mol-% bezogen auf mol Kupfer-Ionen) bevorzugt >0% bis 100%, bevorzugt ≥30% bis ≤70%, noch bevorzugt ≥40% bis ≤60% sowie am meisten bevorzugt etwa 50%.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der zugehörigen Beispiele, in denen - beispielhaft - mehrere Ausführungsbeispiele des erfindungsgemäßen Verfahrens dargestellt sind. Diese sind rein illustrativ zu verstehen.

### Allgemeine Versuchsvorschrift

In ein mit einem Magnetrührstab versehenes, Ofen-getrocknetes Schlenkgefäß (mit 10 mL Fassungsvolumen) wurden Enaminstartmaterial (0.25 mmol) und Cu(OAc)₂ (0.025mmol, 10 Mol-%) eingewogen, gefolgt von der Addition von DCE (1,2-Dichlorethan) (0.5 M), des Nitrils (3.0 Äquiv.) und der 2-Picolinsäure (0.0125 mmol, 5 Mol-%). Anschließend wird die Reaktionsmischung heftig gerührt, das Gefäß kurz evakuiert und anschließend sofort mit Sauerstoff gefüllt, wobei dieser Vorgang (Evakuieren/Befüllen) noch zweimal wiederholt wurde). Das Gefäß wurde anschließend verschlossen und die Reaktionsmischung in einem vorgeheizten Ölbad bei 110 °C für 24 h gerührt. Nach Abkühlen auf Raumtemperatur (Dauer ca. 15 Minuten) wurde die Reaktionsmischung durch ESI-MS, GC-MS und Dünnschichtchromatographie analysiert. Zur Isolierung wurde die Reaktionsmischung mit EtOAc (10 mL) verdünnt und kurz bei Raumtemperatur gerührt. Anschließend wurde durch drei dünne Schichten filtriert (Seesand (0.5 cm), Celite (1.0 cm), Seesand (0.5 cm); zuvor angefeuchtet mit EtOAc). Der verbleibende Rückstand wurde gründlich mit EtOAc gewaschen (4 x 10 mL) und die vereinigten Filtrate wurden im Vakuum eingeengt. Das Rohprodukt wurde in CH₂Cl₂ gelöst, auf Silica adsorbiert und säulenchromatographisch gereinigt (Silica, Gradient von Pentan/EtOAc-Mischungen, wenn nicht anders erwähnt).

### Vergleichsversuche

Zum Vergleich der Wirksamkeit der 2-Picolinsäure mit anderen Hilfssubstanzen wurde eine Versuchsreihe in Anlehnung an die obige Arbeitsvorschrift anhand folgender Reaktion durchgeführt.

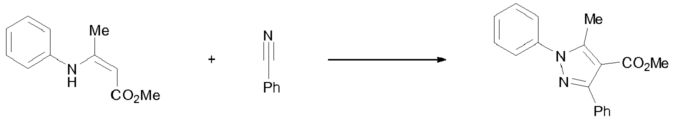

Dabei wurden folgende Reaktionsbedingungen eingestellt: 0.25 mmol Enamin-Edukt, Cu(OAc)₂ (10 Mol-%), PhCN (Anteil siehe Tabelle), Additiv, DCE (0.5 M), 110 °C, 24 h, unter einer O₂ Atmosphäre (1 bar, geschlossenes Gefäß).

Die Versuchsergebnisse (Ausbeuten bestimmt durch GC-Analyse mit Mesitylen als internem Standard) sind in der folgenden Tabelle angegeben:

**Tabelle I**

| Äquiv. PhCN | Additiv (Äquiv.) | Ausbeute (%) |
|---|---|---|
| 1.5 | -- | 22 |
| 1.5 | Pivalinsäure (0.1) | 48 |
| 1.5 | ZnCl₂ (0.1) | 0 |
| 1.5 | Wasser (0.11) | 26 |
| 1.5 | Pivalamid (0.1) | 52 |
| 1.5 | Pivalamid (1.0) | 21 |
| 1.5 | L-Valin (0.1) | 39 |
| 1.5 | Acetamid (0.1) | 37 |
| 1.5 | Benzamid (0.1) | 64 |
| 1.5 | *N*-Methylpivalamid (0.1) | 40 |
| 1.5 | *N*-*tert*Butylpivalamid (0.1) | 54 |
| 1.5 | *N*-Phenylpivalamid (0.1) | 51 |
| 1.5 | *N*-Methoxypivalamid (0.1) | 65 |
| 1.5 | *N*-Pivaloyloxypivalamid (0.1) | 27 |
| 1.5 | 9*H*-4,5-Diazafluoren-9-on (0.1) | 28 |
| 1.5 | 2-Pyrrolidinon (0.1) | 53 |
| 1.5 | *N*-Methyl-2-pyrrolidinon (0.1) | 38 |
| 1.5 | *N*-Methoxy benzamid (0.1) | 28 |
| 1.5 | Oxazolidin-2-on (0.1) | 45 |
| 1.5 | Piperidin-2-on (0.1) | 52 |
| 1.5 | *N*-Methoxypivalamid (1.0) | 44 |
| 1.5 | *N*-Methoxypivalamid (0.5) | 48 |
| 1.5 | *N*-Methoxypivalamid (0.25) | 45 |
| 1.5 | *N*-Methoxypivalamid (0.05) | 67 |
| 1.5 | *N*-Methoxypivalamid (0.05) | 16 |
| 1.5 | *N*-Methoxypivalamid (0.05) | 32 |
| 3.0 | *N*-Methoxypivalamid (0.05) | 69 |
| 3.0 | *N*-Methoxypivalamid (0.05) | 35 |
| 3.0 | *N*-Pyridin-2-ylbenzamid (0.05) | 27 |
| 3.0 | 2-Acetamido benzoesäure (0.05) | 69 |
| 3.0 | Pivalinsäure (0.05) | 40 |
| 3.0 | Thiophen-2-carbonsäure (0.05) | 59 |
| 3.0 | Pyridin (0.05) | 39 |
| 3.0 | DIPEA (0.05) | 43 |
| 3.0 | Et₃N (0.05) | 35 |
| 3.0 | *N*-Methoxypicolinamid (0.05) | 23 |
| 3.0 | 1H-Imidazol-2-carbonsäure (0.05) | 20 |
| 3.0 | 1*H*-Pyrazol-3-carbonsäure (0.05) | 33 |
| 3.0 | DL-Prolin (0.05) | 14 |
| | | |
| 3.0 | 2-Picolinsäure(0.05) | 84 |

Man sieht die deutlich erhöhte Ausbeute bei Verwendung von Picolinsäure im Vergleich zu den anderen Additiven.

Weiterhin wurden, jeweils nach der allgemeinen Versuchsvorschrift, die folgenden Pyrazole synthetisiert:

### Methyl 5-methyl-1,3-diphenyl-1H-pyrazol-4-carboxylat

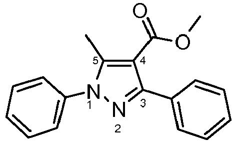

Gelblicher Feststoff.
Ausbeute unter Reaktionsbedingungen: 49.6 mg, 0.17 mmol, 68%.
**R_{f}** (Pentan/EtOAc 90:10): 0.21;
**¹H NMR** (300 MHz, CDCl3): δ = 7.72-7.63 (m, 2H, Hₐᵣₒₘ), 7.56-7.35 (m, 8H, Hₐᵣₒₘ), 3.77 (s, 3H, CH₃), 2.59 (s, 3H, CH₃);
**¹³C NMR** (75 MHz, CDCl3): δ = 164.7 (CO), 153.7 (Cₐᵣₒₘ), 145.0 (Cₐᵣₒₘ), 138.8 (Cₐᵣₒₘ), 133.1 (Cₐᵣₒₘ), 129.4 (CHₐᵣₒₘ), 129.3 (CHₐᵣₒₘ), 128.8 (CHₐᵣₒₘ), 128.4 (CHₐᵣₒₘ), 127.8 (CHₐᵣₒₘ), 125.9 (CHₐᵣₒₘ), 110.4 (Cₐᵣₒₘ), 51.2 (CH₃), 12.9 (CH₃);
***GC-MS:t_{R} (50_20)*:** 15.6 min;
***EI-MS: m*/*z (%) = 292 (100), 262 (21), 261 (100), 260 (22), 259 (20), 118 (15), 77 (52), 51 (14);***
***Exact Mass ESI-MS:*** berechnetes m/z für [C₁₈H₁₆N₂NaO₂]⁺: 315.1104, found: 315.1095; ***ATR-FTIR (cm⁻¹):*** 2948, 2406, 2236, 1712, 1595, 1529, 1500, 1453, 1428, 1382, 1327, 1307, 1246, 1183, 1148, 1088, 994, 770, 696, 655.

### Methyl 5-methyl-1-phenyl-3-(m-tolyl)-1H-pyrazol-4-carboxylat

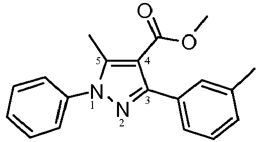

Gelblicher Feststoff
Ausbeute unter Reaktionsbedingungen: 30.6 mg, 0.10 mmol, 40%.
**R_{f}** (Pentan/EtOAc 90:10): 0.23;

### Methyl 3-(4-ethoxyphenyl)-5-methyl-1-phenyl-1H-pyrazol-4-carboxylat

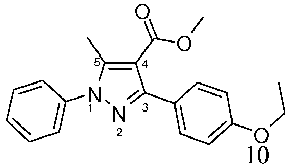

Gelblicher Feststoff.
Ausbeute unter Reaktionsbedingungen: 29.4 mg, 0.09 mmol, 35%. **R_{f}** (Pentan/EtOAc 90:10): 0.13;

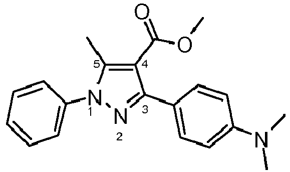

### Methyl 3-(4-(dimethylamino)phenyl)-5-methyl-1-phenyl-1H-pyrazol-4-carboxylat

Gelber Feststoff.
Ausbeute unter Reaktionsbedingungen: 16.8 mg, 0.05 mmol, 20%. **R_{f}** (Pentan/EtOAc 90:10): 0.13;

### Methyl 3-(4-acetylphenyl)-5-methyl-1-phenyl-1H-pyrazol-4-carboxylat

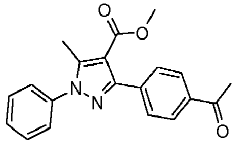

Gelber Feststoff
Ausbeute unter Reaktionsbedingungen: 66.6 mg, 0.20 mmol, 80%
**R_{f}** (Pentan/EtOAc 90:10): 0.04;

### Methyl 5-methyl-1-phenyl-3-(3-(trifluoromethyl)phenyl)-1H-pyrazol-4-carboxylat

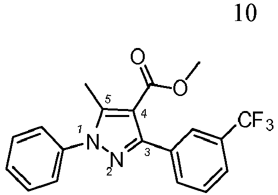

Gelber Feststoff
Ausbeute unter Reaktionsbedingungen: 69.9 mg, 0.19 mmol, 78%
**R_{f}** (Pentan/EtOAc 90:10): 0.22;

### Methyl 3-(2-fluorophenyl)-5-methyl-1-phenyl-1H-pyrazol-4-carboxylat

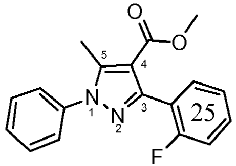

### Gelber Feststoff

Ausbeute unter Reaktionsbedingungen: 15.5 mg, 0.05 mmol, 20%
**R_{f}** (Pentan/EtOAc 90:10): 0.19;

### Methyl 3-(furan-2-yl)-5-methyl-1-phenyl-1H-pyrazol-4-carboxylat

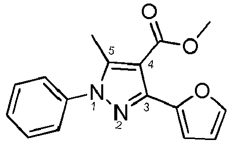

Gelber Feststoff
Ausbeute unter Reaktionsbedingungen: 46.2 mg, 0.16 mmol, 66% **R_{f}** (Pentan/EtOAc 90:10): 0.15;

### Methyl 3,5-dimethyl-1-phenyl-1H-pyrazol-4-carboxylat

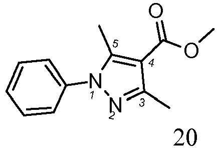

### Gelblicher Feststoff

Ausbeute unter Reaktionsbedingungen: 17.4 mg, 0.08 mmol, 30% **R_{f}** (Pentan/EtOAc 90:10): 0.17;

### Methyl 3-ethyl-5-methyl-1-phenyl-1H-pyrazol-4-carboxylat

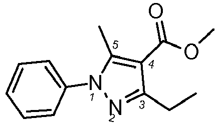

### Gelber Feststoff

Ausbeute unter Reaktionsbedingungen: 19.5 mg, 0.08 mmol, 32% R_{f} (Pentan/EtOAc 90:10): 0.26;

### Methyl 3-(4-fluorophenyl)-5-methyl-1-phenyl-1H-pyrazol-4-carboxylat

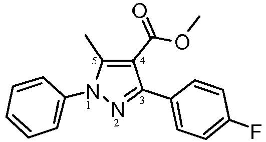

### Weißer Feststoff

Ausbeute unter Reaktionsbedingungen: 54.3 mg, 0.17 mmol, 70%
**R_{f}** (Pentan/EtOAc 90:10): 0.22;

### Methyl 3-(4-fluorophenyl)-5-methyl-1-(o-tolyl)-1H-pyrazol-4-carboxylat

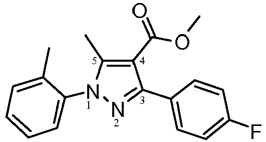

### Gelblicher Feststoff

Ausbeute unter Reaktionsbedingungen: 54.3mg, 0.17 mmol, 67%
**R_{f}** (Pentan/EtOAc 90:10): 0.23;

### Methyl 3-(4-fluorophenyl)-5-methyl-1-(m-tolyl)-1H-pyrazol-4-carboxylat

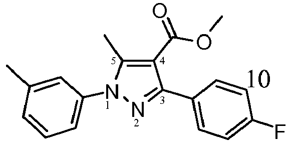

### Gelber Feststoff

Ausbeute unter Reaktionsbedingungen: 50.3 mg, 0.16 mmol, 62% **R_{f}** (Pentan/EtOAc 90:10): 0.23;

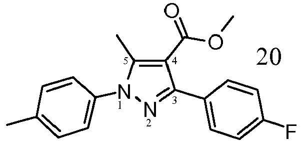

### Methyl 3-(4-fluorophenyl)-5-methyl-1-(p-tolyl)-1H-pyrazol-4-carboxylat

### Gelber Feststoff

Ausbeute unter Reaktionsbedingungen: 55.8 mg, 0.17 mmol, 69% **R_{f}** (Pentan/EtOAc 90:10): 0.20;

### Methyl 1-(4-ethoxyphenyl)-3-(4-fluorophenyl)-5-methyl-1H-pyrazol-4-carboxylat

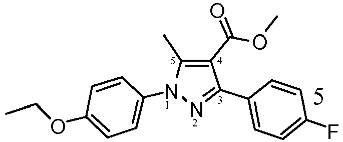

### Gelber Feststoff

Ausbeute unter Reaktionsbedingungen: 65.5 mg, 0.18 mmol, 74%
**R_{f}** (Pentan/EtOAc 90:10): 0.17;

### Methyl 1-(4-(dimethylamino)phenyl)-3-(4-fluorophenyl)-5-methyl-1H-pyrazol-4-carboxylat

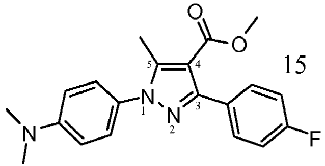

### Gelbes Öl

Ausbeute unter Reaktionsbedingungen: 13.2 mg, 0.04 mmol, 15%
**R_{f}** (Pentan/EtOAc 90:10): 0.11;

### Methyl 1,3-bis(4-fluorophenyl)-5-methyl-1H-pyrazol-4-carboxylat

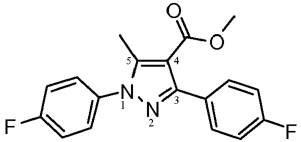

### Weißer Feststoff

Ausbeute unter Reaktionsbedingungen: 48.5 mg, 0.15 mmol, 59%
**R_{f}** (Pentan/EtOAc 90:10): 0.22;

### Methyl 1-(4-(ethoxycarbonyl)phenyl)-3-(4-fluorophenyl)-5-methyl-1H-pyrazol-4-carboxylat

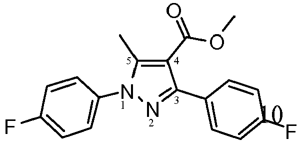

### Weißer Feststoff

Ausbeute unter Reaktionsbedingungen: 59.2 mg, 0.15 mmol, 62%
**R_{f}** (Pentan/EtOAc 90:10): 0.13;

### Methyl 3-(4-fluorophenyl)-5-methyl-1-(2-(trifluoromethyl)phenyl)-1H-pyrazol-4-carboxylat (3s)

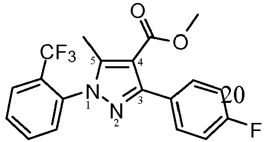

### Gelbes Öl

Ausbeute unter Reaktionsbedingungen: 46.5 mg, 0.12 mmol, 49% **R_{f}** (Pentan/EtOAc 90:10): 0.19;

### Methyl 3-(3-fluorophenyl)-5-methyl-1-(4-(trifluoromethyl)phenyl)-1H-pyrazol-4-carboxylat (3t)

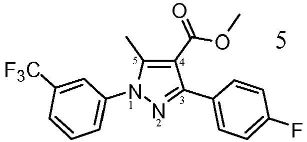

### Weißer Feststoff

Ausbeute unter Reaktionsbedingungen: 56.8 mg, 0.15 mmol, 60%
**R_{f}** (Pentan/EtOAc 90:10): 0.20;

### Methyl 3-(4-fluorophenyl)-5-methyl-1-(4-(trifluoromethyl)phenyl)-1H-pyrazol-4-carboxylat (3u)

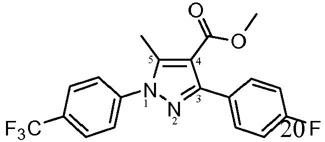

### Gelblicher Feststoff

Ausbeute unter Reaktionsbedingungen: 53.9 mg, 0.14 mmol, 57%
**R_{f}** (Pentan/EtOAc 90:10): 0.19;

### Methyl 3-(4-fluorophenyl)-1,5-dimethyl-1H-pyrazol-4-carboxylat (3v)

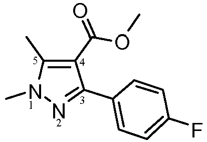

### Weißer Feststoff

Ausbeute unter Reaktionsbedingungen: 26.1 mg, 0.11 mmol, 42%
**R_{f}** (Pentan/EtOAc 90:10): 0.03;

### Methyl 1-butyl-3-(4-fluorophenyl)-5-methyl-1H-pyrazol-4-carboxylat

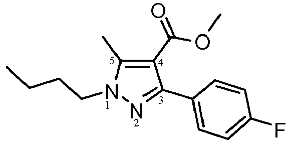

### Gelbes Öl

Ausbeute unter Reaktionsbedingungen: 25.4 mg, 0.09 mmol, 35%
**R_{f}** (Pentan/EtOAc 90:10): 0.19;

### Ethyl 3-(4-fluorophenyl)-1-mesityl-5-methyl-1H-pyrazol-4-carboxylat

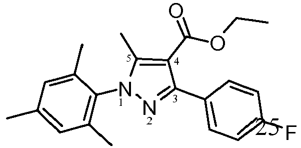

### Gelblicher Feststoff

Ausbeute unter Reaktionsbedingungen: 66.1 mg, 0.18 mmol, 72% **R_{f}** (Pentan/EtOAc 90:10): 0.46;

### Ethyl 1-(2,6-diisopropylphenyl)-3-(4-fluorophenyl)-5-methyl-1H-pyrazol-4-carboxylat

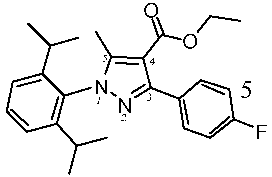

### Gelblicher Feststoff

Ausbeute unter Reaktionsbedingungen: 25.4 mg, 0.06 mmol, 25%
**R_{f}** (Pentan/EtOAc 90:10): 0.53;

### Ethyl 3-(4-fluorophenyl)-1,5-diphenyl-1H-pyrazol-4-carboxylat

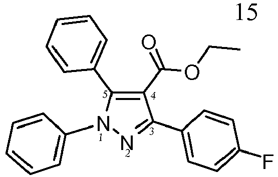

### Gelblicher Feststoff

Ausbeute unter Reaktionsbedingungen: 64.5 mg, 0.17 mmol, 67%
**R_{f}** (Pentan/EtOAc 90:10): 0.34;

### (3-(4-fluorophenyl)-5-methyl-1-phenyl-1H-pyrazol-4-yl)(phenyl)methanon

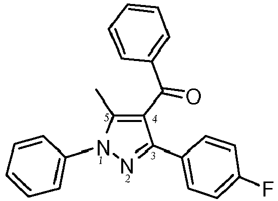

### Weißer Feststoff

Ausbeute unter Reaktionsbedingungen: 16.1 mg, 0.05 mmol, 18%
**R_{f}** (Pentan/EtOAc 90:10): 0.16;

Die einzelnen Kombinationen der Bestandteile und der Merkmale von den bereits erwähnten Ausführungen sind exemplarisch; der Austausch und die Substitution dieser Lehren mit anderen Lehren, die in dieser Druckschrift enthalten sind mit den zitierten Druckschriften werden ebenfalls ausdrücklich erwogen. Der Fachmann erkennt, dass Variationen, Modifikationen und andere Ausführungen, die hier beschrieben werden, ebenfalls auftreten können ohne von dem Erfindungsgedanken und dem Umfang der Erfindung abzuweichen. Entsprechend ist die obengenannte Beschreibung beispielhaft und nicht als beschränkend anzusehen. Das in den Ansprüchen verwendetet Wort "umfassen" schließt nicht andere Bestandteile oder Schritte aus. Der unbestimmte Artikel "ein" schließt nicht die Bedeutung eines Plurals aus. Die bloße Tatsache, dass bestimmte Maße in gegenseitig verschiedenen Ansprüchen rezitiert werden, verdeutlicht nicht, dass eine Kombination von diesen Maßen nicht zum Vorteil benutzt werden kann. Der Umfang der Erfindung ist in den folgenden Ansprüchen definiert und den dazugehörigen Äquivalenten.

## Patentansprüche

1. Verfahren zur Herstellung von Pyrazolen durch Umsetzung von Enaminen und N-haltigen Carbonsäurederivaten in Gegenwart eines Oxidationsmittels, Kupferionen sowie 2-Picolinsäure(derivaten).

2. Verfahren nach Anspruch 1, wobei die N-haltigen Carbonsäurederivate ausgewählt sind aus der Gruppe enthaltend Nitrile, Carbonsäureamide, Amidine, Imidate, Imidoylchloride, abgeleitete protonierte Salze dieser Verbindungen sowie Mischungen daraus.

3. Verfahren nach Anspruch 1 oder 2, wobei die Umsetzung in Gegenwart von Cu(II)-Salzen durchgeführt wird und das Verhältnis von Enamin zu Cu(II)-Salz von ≥1.5:1 bis ≤30:1 (mol Cu(II)-Salz:mol Enamin) beträgt.

4. Verfahren nach einem der Ansprüche 1 bis 3, wobei das Verhältnis von 2-Picolinsäure(derivaten) zu Enamin von ≥0.01% bis ≤10% (mol/mol) beträgt.

5. Verfahren nach einem der Ansprüche 1 bis 4, wobei als N-haltiges Carbonsäurederivat ein Nitril gewählt wird und die Umsetzung in diesem Nitril als Lösemittel durchgeführt wird.

6. Verfahren nach einem der Ansprüche 1 bis 5, wobei das Verhältnis von Enamin zu N-haltigem Carbonsäurederivat von ≥0.2:1 bis ≤150:1 (mol N-haltiges Carbonsäurederivat :mol Enamin) beträgt.
